# EUROPÄISCHE PATENTSCHRIFT

(11) **EP 3 498 230 B1**
(45) Veröffentlichungstag und Bekanntmachung des Hinweises auf die Patenterteilung: **28.10.2020**
(21) Anmeldenummer: 17207776.0
(22) Anmeldetag: 15.12.2017
(51) Int. Cl.: A61F 2/36

(54) **MODULARES ENDOPROTHESEN-SCHAFTSYSTEM MIT ROTATIONSELEMENT**
MODULAR ENDOPROSTHESIS SHAFT SYSTEM WITH ROTATING ELEMENT
SYSTÈME DE TIGE D'ENDOPROTHÈSE MODULAIRE DOTÉ DE L'ÉLÉMENT DE ROTATION

(43) Veröffentlichungstag der Anmeldung: 19.06.2019
(73) Patentinhaber: Waldemar Link GmbH & Co. KG, 22339 Hamburg (DE)
(72) Erfinder: Link, Helmut D., 22397 Hamburg (DE); Borchers, Udo, 22850 Norderstedt (DE); Erb, Gunnar, 21077 Hamburg (DE)
(74) Vertreter: Glawe, Delfs, Moll

(56) Entgegenhaltungen:
- EP-A1- 0 145 641
- EP-A1- 0 359 485
- WO-A1-97/18776
- WO-A2-2007/028832
- US-B1- 7 998 218

## Beschreibung

Die Erfindung betrifft eine Endoprothese für langgestreckte Knochen mit einem modularen Schaftsystem. Das modulare Schaftsystem umfasst mehrere, mittels einer Steckzapfenverbindung zu koppelnde Elemente. Zur Einstellung einer gegenseitigen Verdrehung der Elemente untereinander ist ein Rotationselement vorgesehen, wobei eine damit eingestellte Stellung mittels einer formschlüssig wirkenden Rotationssicherung fixiert ist.

Endoprothesen sind mittels eines Verankerungsteils im Knochen gehaltert und weisen Schäfte auf zum Tragen von Gelenkskomponenten oder zum Ersatz von defekten Knochenteilen. Der jeweilige Schaft kann je nach Anwendungsfall eine beträchtliche Länge erreichen. Um hier mit einer überschaubaren Anzahl von unterschiedlichen Elementen unterschiedliche Längen abdecken zu können, haben sich modulare Systeme bewährt. Diese umfassen mehrere zusammensteckbare Elemente, wobei als Steckverbindungen meist Konuskupplungen vorgesehen sind. Sie umfassen jeweils einen Konuszapfen und eine Konushülse, die im zusammengesteckten Zustand in der Regel hinreichend fest aneinander halten, um axial wirkende Kräfte wie auch Drehkräfte sicher und ohne Verschiebung übertragen zu können.

Bei der Implantation einer solchen Endoprothese ergibt sich mitunter das Bedürfnis, den Schaft in eine bestimmte, anatomisch günstige Orientierung zu drehen. Dies kann erforderlich sein, um einen an einem Ende des Schafts angeordneten Prothesenkopf in eine günstige Richtung zu bringen. Bei bekannten modularen Schaftsystemen (beispielsweise das von der Firma Link, Hamburg, angebotene Megasystem C) können die einzelnen Komponenten des Schafts zwar dank der großartigen Steckverbindung beliebiger Drehorientierung zueinander verbunden werden, aber eine Korrektur der relativen Verdrehung zwischen den einzelnen Komponenten nach dem Verbinden ist schwierig. Ferner ist ein modulares Prothesensystem bekannt (beispielsweise das von der Firma implantcast, Buxtehude, angebotene System MUTARS), bei dem entlang der Schaftachse eine Verschraubung zur Sicherung mittig durchgeführt ist. Durch Lösen dieser Verschraubung können die einzelnen Elemente gegeneinander verdreht werden, und nach Anziehen dieser Verschraubung sind die über eine Passung mit Verzahnung verbundenen Elemente drehgesichert. Die Betätigung setzt aber voraus, dass das obere und untere Ende des Schafts zugänglich ist. Gerade dies ist nach der Implantation häufig nicht mehr der Fall.

In dem Dokument WO 2007/028832 A2 wird ein Endoprothesensatz mit Distraktionsinstrument beschrieben, bei dem sich die Segmente des Endoprothesensatzes nach hoher Belastung wie einer Reoperation leicht trennen lassen.

Der Erfindung liegt die Aufgabe zugrunde, ein verbessertes Schaftsystem für eine Endoprothese bereitzustellen, das diese Nachteile vermeidet.

Die erfindungsgemäße Lösung liegt in den Merkmalen des unabhängigen Anspruchs. Vorteilhafte Weiterbildungen sind Gegenstand der abhängigen Ansprüche.

Bei einem Endoprothesen-Schaftsystem für langgestreckte Knochen, umfassend ein Verankerungsteil und einen Schaft, der aus mittels einer Steckverbindung koppelbaren Modulelementen gebildet ist, wobei eine gegenseitige Verdrehung der Elemente entlang des Schafts einstellbar und mittels einer Rotationssicherung gesichert ist, ist erfindungsgemäß vorgesehen, dass zur Einstellung der Verdrehung ein Rotationsstück als weiteres Modulelement vorgesehen ist, welches in sich abgeschlossen eine Rotationsstelleinrichtung und eine Spanneinrichtung als Sicherung der Rotation aufweist, wobei das Rotationsstück zwei Stirnseiten für die Steckverbindung und einen Mantel aufweist.

Die Erfindung stellt mit dem Rotationsstück ein besonderes Modulelement für den Schaft bereit, das als bauliche Einheit sowohl eine Rotationsverstellung ermöglicht wie auch die Sicherung gegen unerwünschte Verdrehung in einer in sich abgeschlossenen Einheit. Durch die Ausführung als ein Zwischenstück am Schaft kann es somit an einer solchen Stelle im Schaft eingefügt sein, die zweckmäßigerweise während und nach der Implantation gut zugänglich ist (häufig wird dies eine mittlere Position sein). Damit ist eine Verstellbarkeit auch nach der Implantation gewährleistet. Insbesondere bei Endoprothesen, deren Enden nicht zugänglich sind oder deren Positionierung durch dort angeordnete Prothesengelenke erfolgt (beispielsweise bei einem Schaft für den Femur: oben der Kopf einer Hüftprothese und unten die Kondylen einer Knieprothese), ist so eine Ausrichtung in rotatorischer Richtung stets gewährleistet. Ebenso wichtig wie die Fähigkeit zur Verdrehung ist aber auch eine Sicherung gegenüber unbeabsichtigter Weiterverdrehung. Die Erfindung verbindet beide Funktionen in einem Element und ermöglicht so eine räumlich kompakte und vom Zugriff her günstige Kombination von leichter Verstellbarkeit einerseits und starker Sicherung andererseits.

Die Ausführung als modulares Element in einem modularen Schaftsystem erlaubt es der Erfindung, dass erfindungsgemäße Rotationselement auch als Nachrüstung für bestehende Schaftsysteme einzusetzen. Das Anwendungsspektrum erweitert sich damit beträchtlich.

Aus der Sicht des Patienten bietet die Erfindung eine bessere Einstellbarkeit an die individuellen anatomischen Verhältnisse, und ermöglicht dies gegebenenfalls auch nachträglich. Aufwändige Revisionsoperationen können damit vermieden werden.

Zweckmäßigerweise ist das Rotationsstück so ausgeführt, dass es einem Verlängerungsmodulelement des modularen Schaftsystems entspricht. Auf diese Weise kann leicht ein herkömmliches Verlängerungselement gegen das erfindungsgemäße Rotationsstück ausgetauscht werden. Damit fügt sich das Rotationsstück gut in bestehende modulare Schaftsysteme ein.

Zweckmäßigerweise ist die Spanneinrichtung so ausgeführt, dass sie einen Mittelzugriff für ein Betätigungsorgan zum Setzen und/oder Lösen der Sicherung aufweist. Unter einem Mittelzugriff wird hierbei eine Ankoppeleinrichtung für das Betätigungsorgan verstanden, die nicht am Anfang oder Ende des Schafts angeordnet ist. Dank eines solchen Mittelzugriffs kann an dem Schaft auch im implantierten Zustand eine Rotationsverstellung vorgenommen werden, nämlich durch Zugriff von der Seite. Gerade bei einer Prothese mit langem Schaft ist ein solcher seitlicher Zugang in der Regel anatomisch deutlich günstiger.

Vorzugsweise ist eine Hutmutter vorgesehen, die im eingesetzten Zustand einen Innenraum des Rotationsstücks begrenzt. Eine solche Hutmutter ermöglicht zum einen eine Befestigung, und schafft zum anderen mit ihrem Innenraum einen geschützten Bereich. Dort wird somit eine Kammer gebildet, die sich zur Aufnahme von empfindlichen Lagerkomponenten eignet, wie weiter unten noch näher erläutert werden wird.

Ferner bildet die Hutmutter zweckmäßigerweise mit ihrer Außenseite einen Teil des Mantels des Rotationsstücks. Damit kann eine kompakte Bauweise mit geringem Durchmesser erreicht werden, so dass sich die erfindungsgemäße Endoprothese auch zur Anwendung an dünneren Knochen eignet. Vorzugsweise weist die Hutmutter einen glattflächigen, weiter vorzugsweise runden Mantel auf, an dem zweckmäßigerweise Kupplungselemente für ein Spannwerkzeug ausgebildet sind. Der glattflächige Mantel bietet den Vorteil einer minimalen Irritation von umliegenden Gewebe. Das gilt insbesondere dann, wenn der Mantel rund ausgeführt ist. Zweckmäßigerweise sind Kupplungselemente für das Spannwerkzeug in dem Mantel integriert. Auf diese Weise kann bei weiterhin minimaler Irritation umliegenden Gewebes eine sichere Ankopplung des Spannwerkzeugs erreicht werden. Mit Vorteil sind die Kupplungselemente in Umfangsrichtung versetzt angeordnet. Dies ermöglicht ein einfaches Umsetzen des Spannwerkzeugs in das nächste Kupplungselement beim Betätigen, und minimiert so die Anforderungen an die Weite des Zugangs.

Ferner weist die Hutmutter vorzugsweise einen zentralen Durchlass auf, durch den ein Element der Steckverbindung geführt ist. Vorzugsweise ist dieses Element direkt mit einem Lagereinsatz der Rotationstelleinrichtung verbunden. Somit kann dank des zentralen Durchlasses die Kraft von der Steckverbindung direkt zum Lager geführt werden, sodass die Hutmutter selbst außerhalb des eigentlichen Kraftpfads liegt. Vorzugsweise sind der Lagereinsatz und das Element der Steckverbindung kombiniert ausgeführt, und zwar insbesondere einteilig. Dies ermöglicht eine besonders direkte, sichere und spielfreie Kraftübertragung.

Mit Vorteil ist vorgesehen, dass ein Drehlager der Rotationsstelleinrichtung vollständig in dem Rotationsstück angeordnet ist, und zwar vorzugsweise gekammert in dem Innenraum des Rotationsstücks. Besagter Innenraum kann, wie bereits vorstehend erläutert, zweckmäßigerweise geschaffen sein durch den inneren Bereich der Hutmutter. Damit wird ein geschützter Bereich für das Drehlager gebildet. Es ist somit in wirksamer Weise vor Verschmutzung oder Verunreinigungen geschützt. Das Drehlager umfasst zweckmäßigerweise einen Lagerzapfen und eine komplementäre Lagerbuchse, wobei der Lagerzapfen vorzugsweise zylindrisch ist. Die zylindrische Gestaltung bietet den praktischen Vorteil, dass eine axiale Verschiebbarkeit gewährleistet ist unter Beibehaltung der Funktionalität des Drehlagers. Es ist zu sozusagen in das Drehlager noch ein Schiebegelenk integriert. Lagerzapfen und Lagerhülse bilden so vorzugsweise eine spiel-und verklemmungsfreie Passung. Auf diese Weise wird eine hohe und sichere Kraftübertragung gewährleistet, und dennoch eine gute Rotierbarkeit erreicht.

Zweckmäßigerweise ist in dem Innenraum eine auf das Drehlager wirkende Arretierungsscheibe der Sicherungseinrichtung angeordnet. Die Arretierungsscheibe ist zweckmäßigerweise als eine Vielzahnscheibe ausgeführt. Sie greift in das Drehlager ein und blockiert eine weitere Drehung. Zweckmäßigerweise ist die Arretierungsscheibe am Grund der Lagerboxen angeordnet. Sie kann dann auf die Stirnfläche des Lagerzapfens wirken. Vorzugsweise ist dazu an der Stirnfläche des Lagerzapfens eine komplementäre Zahnung angeordnet, mit welcher die Arretierungsscheibe formschlüssig im verriegelten Zustand zusammenwirkt.

Zu diesem Zweck ist die Hutmutter vorzugsweise so ausgeführt, dass sie in einer ersten gespannten Stellung die Arretierungsscheibe auf das Drehlager drückt und es so sperrt, und in einer zweiten offenen Stellung die Arretierungsscheibe vom Drehlager frei kommt, so dass das Drehlager beweglich wird. Somit kann durch eine Betätigung der Hutmutter, insbesondere Verdrehen, das Drehlager gesichert bzw. freigegeben werden.

Bei einer zweckmäßigen Ausführungsform ist das Rotationsstück zweiteilig ausgeführt, mit einem Rotationsteil und einem Distanzteil. Das Rotationsteil umfasst die eigentliche Rotationsstelleinrichtung und die Spanneinrichtung. Das Distanzteil fungiert im Wesentlichen als ein vorzugsweise austauschbares Verlängerungselement. Zweckmäßigerweise ist das Distanzteil an seinem Ende drehfest mit dem Rotationsstück verbunden, und weist an seinem anderen Ende ein Gegenelement der Steckverbindung auf. Vorzugsweise ist das Distanzteil und/oder der Lagereinsatz auswechselbar ausgeführt gegen jeweils andere mit unterschiedlichem Steckverbinder. Somit kann durch Austausch dieser beiden Komponenten das erfindungsgemäße Rotationsstück an andere Steckverbindungen angepasst werden. Damit wird eine wesentlich breitere Anwendbarkeit erreicht.

Die Erfindung wird nachfolgend unter Bezugnahme auf die Zeichnung anhand eines vorteilhaften Ausführungsbeispiels näher erläutert. Es zeigen:

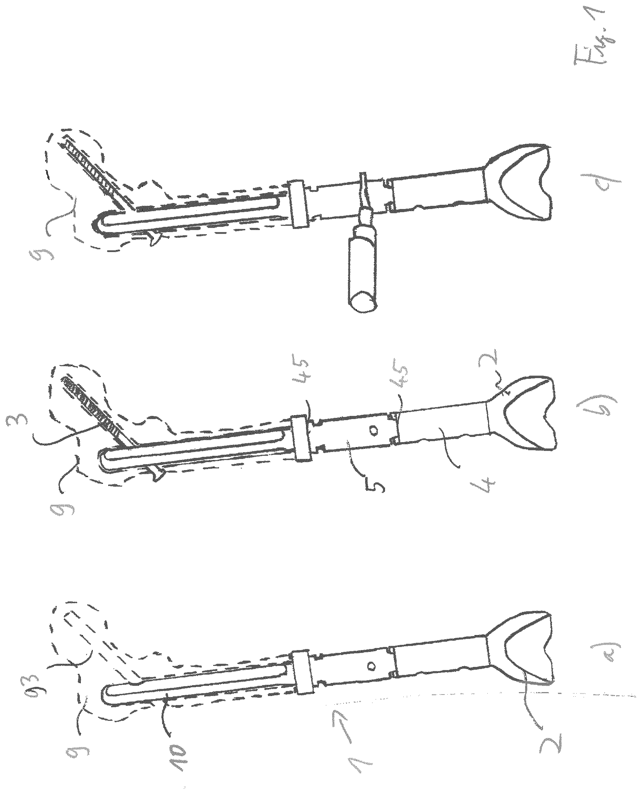

- Fig. 1a-c: Seitenansichten eines modularen Schaftsystems für einen Femur mit Rotationsstück in verschiedenen Phasen;
- Fig.2: eine Seitenansicht sowie eine Schnittansicht zu dem Rotationsstück;
- Fig. 3: eine Explosionsansicht im Schnitt zu dem Rotationsstück;
- Fig. 4: eine Aufsicht auf dem modularen Schaft mit Werkzeug zum Spannen und Verstellen der Rotation; und
- Fig. 5a, b: Darstellungen zum Zustand des modularen Schafts vor und nach der Rotation.

In den Figuren ist ein modulares Schaftsystem zum Einsatz an und teilweisem Ersatz eines Femurs dargestellt.

Das modulare Schaftsystem weist einen Schaft 1 mit einer an seinem unteren Ende angeordneten kondylären Gelenkkomponente 2 einer Kniegelenk-Endoprothese auf. Am oberen Ende ist eine Verankerung 3 im Femurkopf vorgesehen, wobei vorliegend die Verankerung 3 als Spongiosaschraube ausgebildet ist.

Der Schaft 1 ist in Modulbauweise gebildet aus mehreren Modulelementen. Er umfasst im oberen Bereich ein Verankerungsteil 10 zur Verankerung im Markkanal eines oberen Teils des Femurknochens sowie ein modulares Verlängerungsteil 4 im unteren Bereich. An das Verlängerungsteil 4 ist als weiteres Modulelement die Gelenkkomponente 2 angeordnet. Das modulare Verlängerungsteil 4 ist Teil eines Baukastens von verschiedenen Verlängerungsstücken, die unterschiedliche Längen aufweisen können.

Zwischen dem Verankerungsteil 10 und dem Verlängerungsteil 4 ist ein Ausführungsbeispiel für ein erfindungsgemäßes Rotationsstück 5 angeordnet. Es ist über eine Steckverbindung 45 sowohl mit dem Verlängerungsteil 4 wie auch mit dem Verankerungsteil 10 lasttragend verbunden. Eine weitere Steckverbindung kann angeordnet sein zwischen dem Verlängerungsteil 4 und der Gelenkskomponente 2.

Bei der Montage der Prothese muss relativ zum Femurknochen 9 eine bestimmte Winkelposition eingehalten werden. Sie ist im oberen Bereich definiert durch die Ausrichtung des Verankerungselements 3 und im unteren Bereich durch die Orientierung der den oberen Teil einer Kniegelenksprothese bildenden Gelenkkomponente 2. Zuerst wird der Verankerungsteil 10 in die Markhöhle des Femurknochens 9 eingesetzt und es wird ein Aufnahmekanal 93 für das Verankerungselement 3 im Bereich des Kopfes des Femurknochens 9 gebohrt. Die Prothese mit dem Rotationselement 5, dem Verlängerungsteil 4 sowie der Gelenkkomponente 2 wird dann montiert. Im nächsten Schritt wird das Verankerungselement 3 eingesetzt und die Prothese somit bezüglich ihrer Winkelposition gegenüber dem Femurknochen 9 festgelegt. Im weiteren Verlauf der Operation ist es dann erforderlich, die Gelenkkomponente 2 für ihre vorgesehene Verwendung als Element der Kniegelenksprothese auszurichten. Dazu muss deren rotatorische Position angepasst werden. Dies erfolgt in der Weise, indem mit einem geeigneten Werkzeug 8 eine Verdrehung des Rotationsstücks 5 bewirkt wird und dieses in der verdrehten Lage gesichert wird.

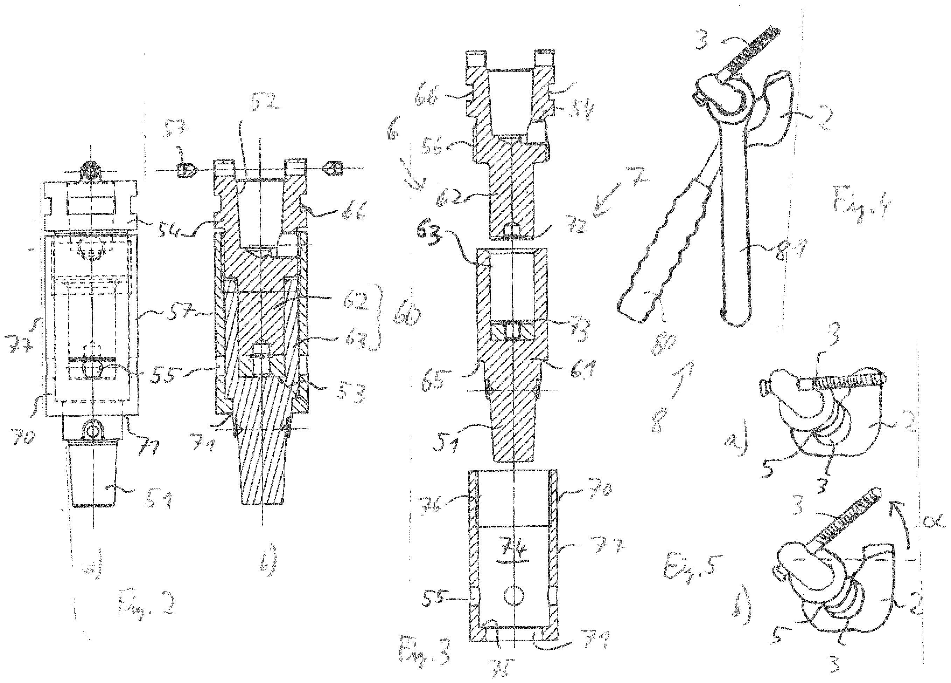

Nachfolgend werden der Aufbau und die Funktionsweise des Rotationsstücks 5 erläutert. Das Rotationsstück 5 umfasst eine Rotationsstelleinrichtung 6 zur Einstellung einer Verdrehung sowie eine Spanneinrichtung 7 zur Sicherung der eingestellten Verdrehung. Gegeneinander verdreht werden hierbei der obere und der untere Bereich des Rotationsstücks 5, so dass im Resultat die Gelenkkomponente 2 relativ zu dem Verankerungsteil 10 rotiert wird. Die durch eine solche Verdrehung eingestellte Position wird auch als "Rotation-Position" bezeichnet. Der Winkel, um den eine solche Verdrehung durchgeführt wird, wird als Rotationswinkel α bezeichnet (vergleiche Figur 5b).

Die Rotationsstelleinrichtung 6 umfasst ein Drehlager 60 bestehend aus einem Lagerzapfen 62 und einer Lagerbuchse 63. Der Lagerzapfen 62 ist an einem Grundkörper 54 angeordnet, während die Lagerbuchse 63 an einem Lagereinsatz 61 angeordnet ist. Der Lagerzapfen 62 ist zylindrisch ausgebildet und die Lagerbuchse 63 ist entsprechend als Passhülse für den Lagerzapfen 62 ausgeführt, so dass dieser spielfrei und verklemmungsfrei darin gelagert ist. Dabei ist der Lagerzapfen 62 in axialer Richtung verschiedentlich zu dem Lagereinsatz 61. An dem Grundkörper 54 ist an der dem Lagerzapfen 62 gegenüberliegenden Seite eine Konushülse 52 der Steckzapfenverbindung 45 ausgebildet. Der Grundkörper 54 bildet somit den oberen Bereich der Rotationsstelleinrichtung 6.

Der Lagereinsatz 61 weist an seiner der Lagerbuchse 63 gegenüberliegenden Seite einen Konuszapfen 51 der Steckzapfenverbindung 45 auf. Der Lagereinsatz 61 bildet somit den unteren Bereich der Rotationsstelleinrichtung 6. Durch gegenseitiges Verdrehen des Lagereinsatzes 61 zu dem Grundkörper 54 kann die gewünschte Rotation im Schaft 1 bewirkt werden. Um dies zu vereinfachen, sind an dem Grundkörper 54 Abflachungen 66 ausgebildet, die als Aufnahmen für einen Werkzeugschlüssel 81 dienen. Damit kann der Grundkörper 54 verdreht werden. Auf diese Weise kann ein gewünschter Rotationswinkel α eingestellt werden.

Um die somit eingestellte Rotationsposition zu sichern, ist die Sicherungseinrichtung 7 vorgesehen. Sie umfasst eine Hutmutter 70 mit einem zentralen Durchlass 71. Sie weist in ihrem Inneren einen Innenraum 74 auf, an dessen Innenwandung an dem dem Durchlass 71 gegenüberliegenden Ende ein Innengewinde 76 vorgesehen ist, welches im eingesetzten Zustand zum Eingriff kommt mit einem am Grundkörper 54 vorgesehenen komplementären Außengewinde 56. Innerhalb der Hutmutter 70 ist eine Kammer 74 gebildet, die in Verbindung steht mit dem Durchlass 71. Im eingesetzten Zustand ist die Hutmutter 70 mit ihrem Durchlass 71 über den Konuszapfen 51 des Lagereinsatzes 61 geschoben, und zwar so weit, bis das Innengewinde 76 in das Außengewinde 56 eingreift. Durch Verdrehen kann die Hutmutter somit angezogen werden, wodurch die Spanneinrichtung 7 betätigt (bzw. durch Verdrehen in Gegenrichtung gelöst) wird. Dabei ist im Bereich des Durchlasses 71 eine Schulter 75 ausgebildet, die auf einen Bund 65 an dem Lagereinsatz 61 wirkt. Durch Anziehen der Hutmutter 70 schraubt sie sich weiter auf das Gewinde 56, 76 und bewegt sich somit in Richtung zu dem Grundkörper 54, wodurch die Schulter 75 auf den Bund 65 des Lagereinsatzes 61 wirkt und diesen mitnimmt. Dieser wird also ebenfalls auf den Grundkörper 54 zubewegt, und zwar so weit, bis eine am Grund der Lagerbuchse 63 drehfest angeordnete Arretierungsscheibe 73 in Vielzahnausführung eingreift in eine entsprechend komplementär geformte Gegenscheibe 72, die an der Spitze des Lagerzapfens 62 drehfest angeordnet ist. Somit greifen durch Festziehen der Hutmutter 70 die Vielzahnscheibe 73 und die Gegenscheibe 72 ineinander, so dass eine weitere Verdrehung der Lagerbuchse 63 relativ zu dem Lagerzapfen 62 verhindert wird. Damit ist die Spanneinrichtung 7 betätigt und sichert das Drehlager 60 gegenüber einer unerwünschten Verdrehung. Durch Losdrehen der Hutmutter 70 kann die Spanneinrichtung 7 entsprechend gelöst werden, um so wieder eine Verdrehung des Drehlagers 60 zu ermöglichen.

Die Hutmutter 70 bildet mit ihrem Innenraum 74 hierbei eine Verkammerung für das Drehlager 60. Es ist damit vor dem Zutritt von Fremdkörpern und Verunreinigungen geschützt, die eine einwandfreie Lagerfunktion sonst gefährden könnten.

Die Hutmutter 70 weist ferner an ihrem Mantel 77 eine Mehrzahl von in Umfangsrichtung versetzten Radialöffnungen auf, die als Koppelelemente 55 für ein Betätigungswerkzeug 80 fungieren. Dieses weist an seinem Kopf entsprechende Vorsprünge (nicht dargestellt) auf, die formschlüssig in die Koppelelemente 55 eingreifen und somit ein Betätigen der Hutmutter 70 durch Verdrehen ermöglichen. Mittels des an die Abflachung 66 des Grundkörpers 54 angesetzten Werkzeugs 80 kann bei Bedarf gegengehalten werden, um so den Schaft im Übrigen kraftfrei zu halten.

Dieser Vorgang des Ansetzens der beiden Werkzeuge 80, 81 ist in Figur 4 dargestellt, um so die Spanneinrichtung 7 zu lösen und ein gegenseitiges Verdrehen der Gelenkkomponente 2 relativ zu dem oberen Schaftteil mit dem Verankerungselement 3 zu ermöglichen. Eine solche Verdrehung um einen Rotationswinkel α ist in den Figuren 5a und 5b dargestellt.

Das in sich geschlossene Rotationsstück 5 mit dem darin angeordneten Rotationselement 6 sowie Spanneinrichtung 7 ermöglicht so auf kompakte Weise eine Verdrehung des Schafts einerseits und eine effektive Sicherung gegen unerwünschte Verstellung andererseits, wobei durch eine Betätigung von der Seite über die Mantelfläche 77 ein handhabungstechnisch günstiger Zugriff ermöglicht ist. Dies wird vorliegend als Mittelzugriff bezeichnet, im Gegensatz zu dem Zugriff über die Enden des Schafts.

Ferner kann bei einer Variante der Erfindung eine zweiteilige Ausführung vorgesehen sein, bei der an dem eigentlichen Rotationsstück 5 ein Distanzteil fest angeordnet ist. Das Distanzteil ist wie ein kurzes Verlängerungsstück ausgeführt mit einer Zapfenverbindung an seinen Stirnseiten, also an seiner einen Seite einen Zapfenkonus und an der gegenüberliegenden Stirnseite eine Zapfenhülse. Es ist mit seinem Zapfenkonus eingesetzt in die Zapfenhülse 52 des Rotationsstücks 5. Über die so geschaffene Steckverbindung 45 sowie durch die als zusätzliche Sicherung fungierenden Madenschrauben 57 ist das Distanzteil fest mit dem Rotationsstück 5 verbunden. Auf diese Weise kann allein durch Austausch des Distanzteils die Länge variiert werden, ohne dass dazu eine konstruktive Änderung am Rotationsstück 5 selbst vorgenommen werden müsste; das Rotationsstück 5 selbst kann unverändert bleiben. Damit ist eine Anpassung des Rotationsstücks 5 an verschiedene Baukastensysteme deutlich erleichtert.

## Patentansprüche

1. Endoprothesen-Schaftsystem für langgestreckte Knochen, umfassend ein Verankerungsteil (10) und einen Schaft (1), der aus mittels einer Steckverbindung (45) koppelbaren Modulelementen gebildet ist, wobei eine gegenseitige Verdrehung der Elemente entlang des Schafts (1) einstellbar und mittels einer Rotationssicherung gesichert ist,
**dadurch gekennzeichnet, dass**
zur Einstellung der Verdrehung ein Rotationsstück (5) als weiteres Modulelement vorgesehen ist, welches in sich abgeschlossen eine Rotationsstelleinrichtung (6) und eine Spanneinrichtung (7) als Sicherung der Rotation aufweist, wobei das Rotationsstück (5) zwei Stirnseiten für die Steckverbindung (45) und einen Mantel (77) aufweist.

2. Endoprothesen-Schaftsystem mit einem modularen Schaftsystem nach Anspruch 1, **dadurch gekennzeichnet, dass** das Rotationsstück (5) als Verlängerungsmodulelement fungiert.

3. Endoprothesen-Schaftsystem nach Anspruch 1 oder 2, **dadurch gekennzeichnet, dass** die Spanneinrichtung (7) einen Mittelzugriff für ein Betätigungsorgan (81) zum Setzen/Lösen der Sicherung aufweist.

4. Endoprothesen-Schaftsystem nach einem der vorangehenden Ansprüche, **dadurch gekennzeichnet, dass** mantelseitig am Rotationsstück (5) ein Zugriff zum Verstellen der Rotationssicherung (7) vorgesehen ist.

5. Endoprothesen-Schaftsystem nach einem der vorangehenden Ansprüche, **dadurch gekennzeichnet, dass** eine Hutmutter (70) vorgesehen ist, die im eingesetzten Zustand einen Innenraum (74) des Rotationsstücks (5) begrenzt, wobei vorzugsweise die Hutmutter (70) mit ihrer Außenseite einen Teil des Mantels (77) des Rotationsstücks (5) bildet.

6. Endoprothesen-Schaftsystem nach Anspruch 5, **dadurch gekennzeichnet, dass** die Hutmutter (70) einen glattflächigen, vorzugsweisen runden Mantel (77) aufweist, an dem Kupplungselemente (55) für ein Spannwerkzeug (81) ausgebildet sind, die vorzugsweise in Umfangsrichtung versetzt angeordnet sind.

7. Endoprothesen-Schaftsystem nach einem der Ansprüche 5 oder 6, **dadurch gekennzeichnet, dass** die Hutmutter (70) einen zentralen Durchlass (71) aufweist, durch den ein Element der Steckverbindung geführt ist, das vorzugsweise direkt mit einem Lagereinsatz (61) der Rotationsstelleinrichtung (6) verbunden ist.

8. Endoprothesen-Schaftsystem nach Anspruch 7, **dadurch gekennzeichnet, dass** der Lagereinsatz (61) und ein Element der Steckverbindung (51) kombiniert ausgeführt sind, vorzugsweise einteilig.

9. Endoprothesen-Schaftsystem nach einem der vorangehenden Ansprüche, **dadurch gekennzeichnet, dass** ein Drehlager (60) der Rotationstelleinrichtung (6) vollständig in dem Rotationsstück (5) angeordnet ist, und zwar vorzugsweise gekammert in einem Innenraum (74).

10. Endoprothesen-Schaftsystem nach Anspruch 9, **dadurch gekennzeichnet, dass** das Drehlager (60) einen vorzugsweise zylindrischen Lagerzapfen (62) und eine komplementäre Lagerbuchse (63) umfasst, wobei vorzugsweise der Lagerzapfen (62) und die Lagerbuchse ( 63) eine spiel- und verklemmungsfreie Passung bilden.

11. Endoprothesen-Schaftsystem nach Anspruch 9 oder 10,
**dadurch gekennzeichnet, dass** eine auf das Drehlager (60) wirkende Arretierungsscheibe (73) der Sicherungseinrichtung in dem Innenraum (74) angeordnet ist, vorzugsweise am Grund der Lagerbuchse (63).

12. Endoprothesen-Schaftsystem nach Anspruch 11, **dadurch gekennzeichnet, dass** die Hutmutter (70) in einer ersten gespannten Stellung die Arretierungsscheibe (73) auf das Drehlager (60) drückt und es so sperrt, und in einer zweiten offenen Stellung die Arretierungsscheibe (73) vom Drehlager (60) frei wird, so dass das Drehlager (60) beweglich ist.

13. Endoprothesen-Schaftsystem nach einem der vorangehenden Ansprüche, **dadurch gekennzeichnet, dass** das Rotationsstück (5) zweiteilig ausgeführt ist, mit einem angesetzten Distanzteil.

14. Endoprothesen-Schaftsystem nach Anspruch 13, **dadurch gekennzeichnet, dass** das Distanzteil an einem Ende drehfest mit dem Rotationsstück (5) verbunden ist und an seinem anderen Ende ein Gegenelement der Steckverbindung (45) trägt.

15. Endoprothesen-Schaftsystem nach Anspruch 14, **dadurch gekennzeichnet, dass** das Distanzteil und/oder der Lagereinsatz (60) auswechselbar sind gegen jeweils andere mit unterschiedlichem Steckverbinder.

## Claims

1. An endoprosthesis shaft system for elongated bones, comprising an anchoring part (10) and a shaft (1), which is formed from a module elements that can be coupled by means of a plug-in connection (45), wherein a mutual rotation of the elements along the shaft (1) is adjustable and is blocked by means of an anti-rotation mechanism, **characterized in that** a rotation piece (5) is provided as a further module element for adjusting the rotation, which has a self-contained rotation adjustment device (6) and a clamping device (7) as a mechanism for blocking the rotation, wherein the rotation piece (5) has two end faces for the plug-in connection (45) and a sleeve (77).

2. The endoprosthesis shaft system having a modular shaft system according to claim 1, **characterized in that** the rotation piece (5) acts as an extension module element.

3. The endoprosthesis shaft system according to claim 1 or 2, **characterized in that** the clamping device (7) has a central grip for an actuation element (81) for setting/releasing the mechanism.

4. The endoprosthesis shaft system according to any of the preceding claims, **characterized in that** a grip for adjusting the anti-rotation mechanism (7) is provided on the sleeve side on the rotation piece (5).

5. The endoprosthesis shaft system according to any of the preceding claims, **characterized in that** a cap nut (70) is provided, which, when installed, limits an interior (74) of the rotation piece (5), wherein preferably the cap nut (70) forms with its exterior a portion of the sleeve (77) of the rotation piece (5).

6. The endoprosthesis shaft system according to claim 5, **characterized in that** the cap nut (70) has a smooth, preferably round sleeve (77) on which coupling elements (55) for a clamping tool (81) are formed, which preferably are arranged offset in circumferential direction.

7. The endoprosthesis shaft system according to any of claims 5 or 6, **characterized in that** the cap nut (70) has a central passage (71), through which an element of the plug-in connection is guided, which is preferably directly connected to a bearing insert (61) of the rotation adjustment device (6).

8. The endoprosthesis shaft system according to claim 7, **characterized in that** the bearing insert (61) and an element of the plug-in connection (51) are designed combined, preferably in one piece.

9. The endoprosthesis shaft system according to any of the preceding claims, **characterized in that** a pivot bearing (60) of the rotation adjustment device (6) is arranged completely in the rotation piece (5), specifically preferably chambered in an interior (74).

10. The endoprosthesis shaft system according to claim 9, **characterized in that** the pivot bearing (60) comprises a preferably cylindrical bearing pin (62) and a complementary bearing bush (63), wherein preferably the bearing pin (62) and the bearing bush (63) form a play-free and clamp-free fit.

11. The endoprosthesis shaft system according to claim 9 or 10, **characterized in that** a locking disk (73) of the safety mechanism acting on the pivot bearing (60) is arranged in the interior (74), preferably on the base of the bearing bush (63).

12. The endoprosthesis shaft system according to claim 11, **characterized in that** the cap nut (70) in a first clamped position presses the locking disk (73) onto the pivot bearing (60) and thus blocks it, and in a second open position the locking disk (73) is free from the pivot bearing (60), so that the pivot bearing (60) is moveable.

13. The endoprosthesis shaft system according to any of the preceding claims, **characterized in that** the rotation piece (5) is designed in two parts, with an inserted distance part.

14. The endoprosthesis shaft system according to claim 13, **characterized in that** the distance part is rotationally fixed to the rotation piece (5) on one end and bears a mating element of the plug-in connection (45) on its other end.

15. The endoprosthesis shaft system according to claim 14, **characterized in that** the distance part and/or the bearing insert (60) can be replaced with respective others having different connectors.

## Revendications

1. Système de manche d'endoprothèse pour des os allongés, qui comprend une partie d'ancrage (10) et un manche (1) qui est formé d'éléments modulaires couplés au moyen d'une liaison par fiche (45), dans lequel une torsion mutuelle des éléments le long du manche (1) est réglable et sécurisée au moyen d'une sécurisation de rotation,
**caractérisé en ce que**
pour régler la torsion est prévue une pièce de rotation (5) en tant qu'autre élément modulaire, lequel présente inclus en lui-même un dispositif de réglage de rotation (6) et un dispositif de serrage (7) en tant que sécurisation de la rotation,
dans lequel la pièce de rotation (5) présente deux faces frontales pour la liaison par fiche (45) et une gaine (77).

2. Système de manche d'endoprothèse avec un système de manche modulaire selon la revendication 1, **caractérisé en ce que** la pièce de rotation (5) sert d'élément modulaire d'extension.

3. Système de manche d'endoprothèse selon la revendication 1 ou 2, **caractérisé en ce que** le dispositif de serrage (7) présente une prise centrale pour un organe d'actionnement (81) afin de mettre/défaire la sécurisation.

4. Système de manche d'endoprothèse selon l'une des revendications précédentes, **caractérisé en ce qu'**une prise est prévue du côté de la gaine contre la pièce de rotation (5) pour ajuster la sécurisation de rotation (7).

5. Système de manche d'endoprothèse selon l'une des revendications précédentes, **caractérisé en ce qu'**est prévu un écrou borgne (70) qui à l'état inséré délimite un espace intérieur (74) de la pièce de rotation (5), dans lequel de préférence l'écrou borgne (70) forme avec son côté extérieur une partie de la gaine (77) de la pièce de rotation (5).

6. Système de manche d'endoprothèse selon la revendication 5, **caractérisé en ce que** l'écrou borgne (70) présente une gaine (77) de surface lisse, de préférence ronde contre laquelle sont formés des éléments de couplage (55) pour un outil de serrage (81) qui de préférence sont agencés en décalage dans la direction circonférentielle.

7. Système de manche d'endoprothèse selon l'une des revendications 5 ou 6, **caractérisé en ce que** l'écrou borgne (70) présente un passage (71) central à travers lequel est guidé un élément de la liaison par fiche, lequel est de préférence directement relié à une garniture de montage (61) du dispositif de réglage de rotation (6).

8. Système de manche d'endoprothèse selon la revendication 7, **caractérisé en ce que** la garniture de montage (61) et un élément de la liaison par fiche (51) sont conçus de façon combinée, de préférence en une seule pièce.

9. Système de manche d'endoprothèse selon l'une des revendications précédentes, **caractérisé en ce qu'**un palier de pivotement (60) du dispositif de réglage de rotation (6) est entièrement agencé dans la pièce de rotation (5), à savoir de préférence enfermé dans un espace intérieur (74).

10. Système de manche d'endoprothèse selon la revendication 9, **caractérisé en ce que** le palier de pivotement (60) comprend un tourillon (62) de préférence cylindrique et un coussinet (63) complémentaire, dans lequel le tourillon (62) et le coussinet (63) forment de préférence une adaptation sans jeu et sans serrage.

11. Système de manche d'endoprothèse selon la revendication 9 ou 10, **caractérisé en ce qu'**un disque d'arrêt (73) du dispositif de sécurisation qui agit sur le palier de pivotement (60) est agencé dans l'espace intérieur (74), de préférence contre la base du coussinet (63).

12. Système de manche d'endoprothèse selon la revendication 11, **caractérisé en ce que** dans une première position tendue l'écrou borgne (70) appuie le disque d'arrêt (73) contre le palier de pivotement (60) et ainsi le bloque, et dans une seconde position ouverte le disque d'arrêt (73) est libéré du palier de pivotement (60) de sorte que le palier de pivotement (60) soit mobile.

13. Système de manche d'endoprothèse selon l'une des revendications précédentes, **caractérisé en ce que** la pièce de rotation (5) est conçue en deux parties, avec une partie de distance rapportée.

14. Système de manche d'endoprothèse selon la revendication 13, **caractérisé en ce que** la partie de distance est reliée à une extrémité en rotation solidaire avec la pièce de rotation (5) et porte à son autre extrémité un contre-élément de la liaison par fiche (45).

15. Système de manche d'endoprothèse selon la revendication 14, **caractérisé en ce que** la partie de distance et/ou le palier de pivotement (60) sont remplaçables par respectivement d'autres avec une fiche de liaison différente.
